(19) Europäisches Patentamt
European Patent Office
Office européen des brevets

(11) EP 3 827 818 A1

(12) **EUROPEAN PATENT APPLICATION**

(43) Date of publication:
**02.06.2021 Bulletin 2021/22**

(21) Application number: **19460062.3**

(22) Date of filing: **26.11.2019**

(51) Int Cl.:
*A61K 31/164* (2006.01)   *A61K 31/197* (2006.01)
*A61K 31/505* (2006.01)   *A61K 31/728* (2006.01)
*A61K 9/00* (2006.01)   *A61K 9/08* (2006.01)
*A61K 47/02* (2006.01)   *A61P 27/04* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicant: **Warszawskie Zaklady Farmaceutyczne
Polfa S.A.
01-207 Warszawa (PL)**

(72) Inventors:
- **Kubisiak, Marcin
  02-315 Warszawa (PL)**
- **Ratajczak, Tomasz
  02-315 Warszawa (PL)**

- **Rochowczyk, Anna
  02-315 Warszawa (PL)**
- **Kopec, Dorota
  02-315 Warszawa (PL)**
- **Popielska, Iwona
  02-315 Warszawa (PL)**
- **Magnuszewska, Monika
  02-315 Warszawa (PL)**
- **Malik, Katarzyna
  02-315 Warszawa (PL)**

(74) Representative: **Obrycki, Pawel Andrzej
Zaklady Farmaceutyczne Polpharma SA
UI. Pelplinska 19
83-200 Starogard Gdanski (PL)**

(54) **OPHTHALMIC COMPOSITION**

(57)      The application refers to an ophthalmic composition comprising: 0.001 to 2 % by weight of hyaluronic acid or an ophthalmologically acceptable salt thereof; 0.5 to 5 % by weight of ectoine or an ophthalmologically acceptable salt or derivative thereof; 0.5 to 5 % by weight of a compound selected from the group consisting of panthenol, pantothenic acid, an ophthalmologically acceptable salt of pantothenic acid, and combinations thereof; and an amount of water up to 100% by weight of the composition. The application also refers to the above composition for use in the prevention and/or treatment of an ophthalmological pathology selected from the group consisting of dry eye syndrome, Sjogren syndrome, inflammation of the conjunctiva, irritation, itchiness, burning and allergic reactions of the eye, and related conditions.

Synergic effect on viscosity for addition of dexpanthenol in compositions 72HED091, 51HED0819_5 and 73HED0919

| Compound | 87HED1119 | 86HED1119 | 87HED1119 | 71HED0919 | 72HED0919 | 51HED0819_5 | 73HED0919 |
|---|---|---|---|---|---|---|---|
| | % w/v | % w/v | % w/v | % w/v | % w/v | % w/v | % w/v |
| *Sodium hyaluronate IV=0.95 m³/kg* | 0 | 0 | 0 | 0.5 | 0.5 | 0.5 | 0.5 |
| *Ectoine* | 0 | 0 | 0 | 2 | 2 | 2 | 2 |
| *Dexpanthenol* | 1 | 2 | 3 | 0 | 1 | 2 | 3 |
| *Sodium chloride* | 0.44 | 0.44 | 0.44 | 0.44 | 0.44 | 0.44 | 0.44 |
| *HCl or NaOH pH 6.3* | q.s. | q.s. | q.s. | q.s. | q.s. | q.s. | q.s. |
| *Water* | Ad 1 mL | Ad 1 mL | Ad 1 mL | Ad 1 mL | Ad 1 mL | Ad 1 mL | Ad 1 mL |

Figure 9

EP 3 827 818 A1

## Description

## Technical Field

The present invention relates to the field of compositions for the prevention and/or treatment of ophthalmic pathologies, in particular for dry eye syndrome.

## Background Art

Dry eye syndrome, also known as dry eye or dry keratoconjunctivitis (KCS), is a condition for which the amount of tears produced by the lacrimal glands is insufficient or tear film evaporates excessively. The reduced flow of tears can result in the formation of only an inadequate or no tear film on the surface of the eye. The tear film acts inter alia as a slip or lubricating agent between the eyelid and the surface of the eye. As a result of the absence of or inadequate tear film, the epithelial layers can suffer from considerable trauma. This results in discomfort in the eye, which is accompanied by itching, tingling and / or burning sensations.

Eye dryness can result from an abnormality of the lacrimal glands, inflammation of the eyelids, ocular inflammation due to an allergy, refractive surgery (including laser), a deficiency of certain lipids in the eye, daily diet, prolonged contact lens wearing, hormonal alteration, autoimmune disease, or secondary effects of certain drugs.

In the treatment of dry eye, the application of saline or artificial tears, also called lubricating or moistening eye drops, is sometimes recommended. Artificial tears of low to medium viscosity are usually based on polyvinyl alcohols or cellulosic derivatives. These remedies provide no more than some local relief for a short time and, besides, they have the disadvantage of changing the cornea surface decreasing its density (Wegener, A. R., Meyer, L. M. & Schonfeld, C. L. Effect of viscous agents on corneal density in dry eye disease. J. Ocul. Pharmacol. Ther. 31, 504-508 (2015).

Eye drops containing hyaluronic acid, natural biopolymer possessing excellent viscoelasticity, high moisture retention capacity and hygroscopic properties, are widely used in the context of the treatment of dry eye. Hyaluronic acid is used in eye drops for the hydration of the cornea. It forms a transparent, lubricating and wetting film on the surface of the eye, protecting the cornea against drying. These properties allow effective relief of clinical signs. However, prior art eye drops containing hyaluronic acid have the problem of limited action time, forcing the patient to repeat the instillations frequently. Moreover, it is known that administration of hyaluronic acid in the eye produces a slight discomfort which lasts from a few seconds to a few minutes after instillation and that is often accompanied by a slight blur in the vision.

In conclusion, hyaluronic acid eye drops are more effective for mild to moderate eye dryness than saline or artificial tears based on polyvinyl alcohols or cellulosic derivatives. Patients with severe drought may also find some relief but are generally forced to turn to different products containing further active ingredients such as immunosuppressants (e.g. ciclosporin) or anti-inflammatories (e.g. lifitegrast). Use of these further active ingredients, however, may have undesired side effects, particularly when used during prolonged time.

The prior art has provided compositions which combine hyaluronic acid with further ingredients in order to improve the treatment of dry eye. However, so far difficulties have been encountered for developing a convenient solution that overcomes the drawbacks mentioned above.

Thus, there exists the need to provide improved compositions for the treatment of dry eye syndrome and related pathologies.

## Summary of Invention

The inventors have developed an ophthalmic composition for the treatment of ophthalmic pathologies, in particular dry eye syndrome, that overcomes the drawbacks mentioned above. Said composition comprises a combination of hyaluronate with ectoine and panthenol which achieves multiple advantages.

The inventors have found that the triple combination of hyaluronate with ectoine and panthenol shows surprisingly high water binding capacity, thus improving moistening of the eye. The water binding capacity of the triple combination is substantially higher than what would have been expected according to the water retention capacity of the individual components or combination of the components taken two by two, thus showing a synergic effect.

A first aspect of the invention thus refers to an ophthalmic composition comprising: 0.001 to 2 % by weight of hyaluronic acid or an ophthalmologically acceptable salt thereof, 0.5 to 5.00% by weight of ectoine or an ophthalmologically acceptable salt or derivative thereof, 0.5 to 5 % by weight of a compound selected from the group consisting of panthenol, pantothenic acid, an ophthalmologically acceptable salt of pantothenic acid, and combinations thereof, and an amount of water up to 100% by weight of the composition. In addition to the increased water binding capacity, the composition of the invention also has a surprisingly high dynamic viscosity, which ensures that the composition adheres to the surface of the eye and remains there for a long time. The viscosity, however, is not too high so as to produce discomfort to the user or to cause occlusion of lacrimal duct when applying to the eye (or after). Advantageously, the viscosity of the invention rapidly drops by applying shear stress, avoiding damage to corneal surface. The triple combination of the composition of the invention conveniently balances effectiveness with remanence and comfort. Generally, the remanence or persistence of a composition is defined as the time during which said composition continues to exert its action, in the absence of repetition of the application. In the context of the invention, the remanence of a composition can be evaluated by measuring the thickness of the tear film. Tear Film Thickness may be measured by High Resolution Optical Coherence tomography.

Moreover, the inventors have found that the presence of dexpanthenol in the composition of the invention unexpectedly results in decreased surface tension. This is very important because the tear film thickness increases exponentially with drop of the surface tension (Creech JL, Do LT, Fatt I, Radke CJ. In vivo tear-film thickness determination and implications for tear-film stability. Curr Eye Res 1998; 17: 1058-1066). In addition, the clinical studies revealed the tears' surface tension is increased in patients with dry eye syndrome (J. M. Tiffany, N. Winter & G Bliss (1989) Tear film stability and tear surface tension, Current Eye Research, 8:5, 507-515).

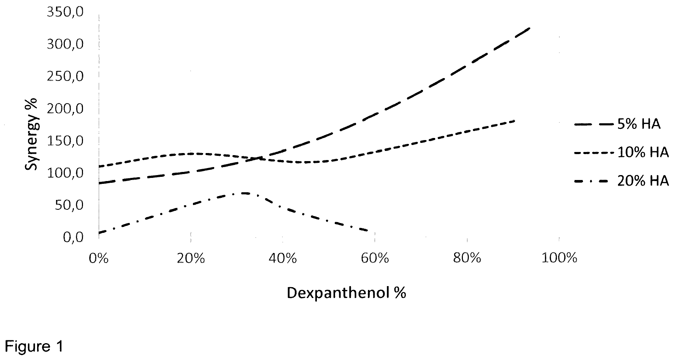

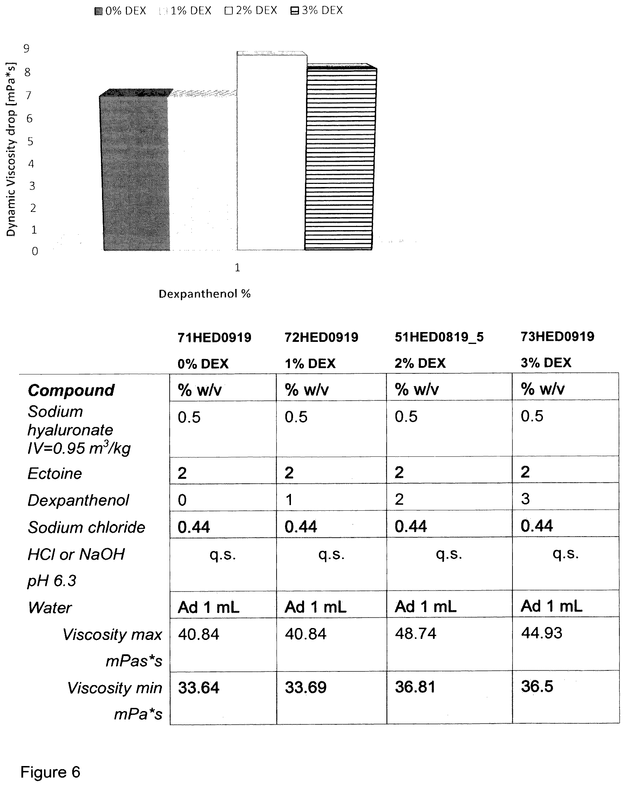

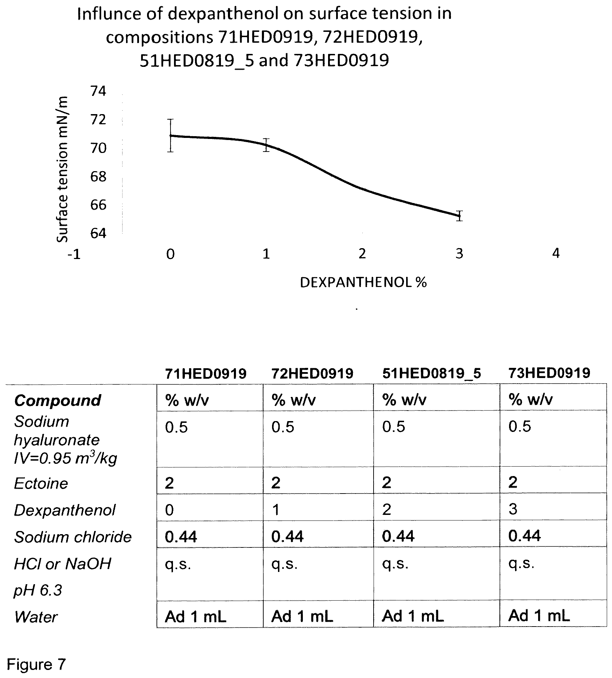

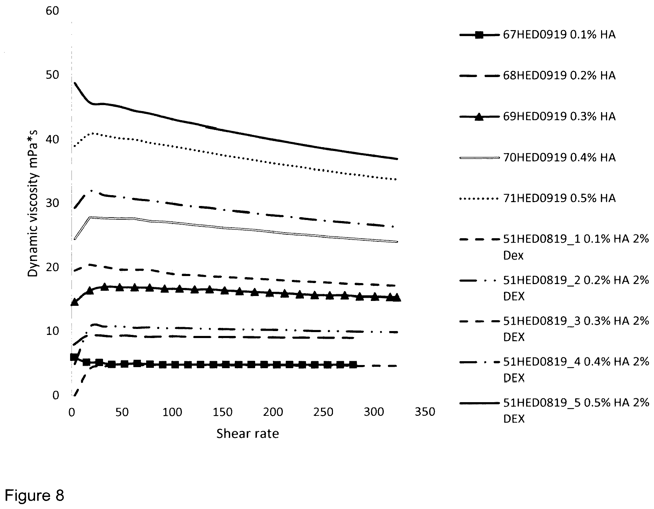

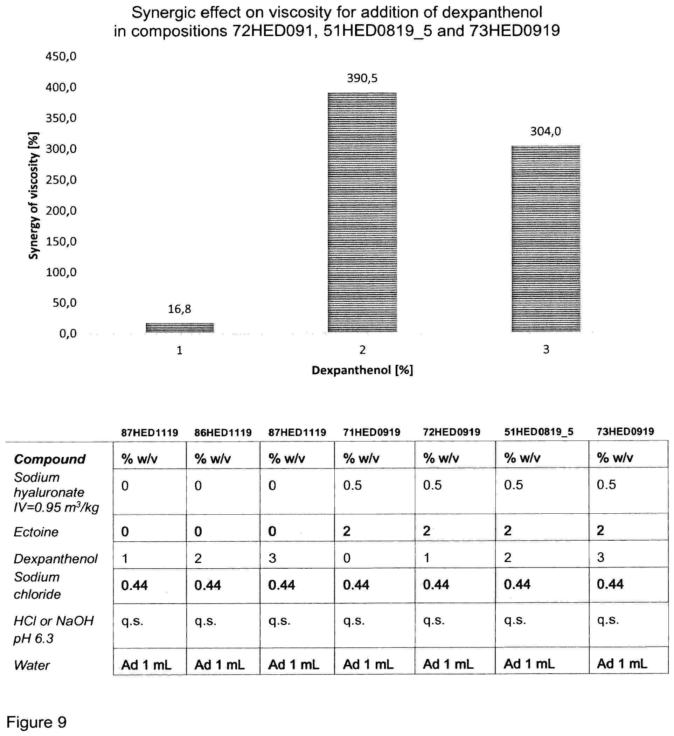

The advantageous effects mentioned above are demonstrated for the composition of the invention in the examples disclosed in the present application. In particular, synergic water binding capacity is shown in figure 1, synergic viscosity is shown in figure 9, reduced surface tension is shown in figure 7, increased dynamic viscosity is shown in figure 8, while dynamic viscosity drop is shown in figure 6.

Finally, the present composition is highly effective for treating dry eye and related condition preferably without the need of additional active ingredients or additional excipients or carriers, and requires no preservatives. The absence of preservatives and additional active agents, excipients or carriers guarantees lack of undesired side effects, such as allergic reactions, secondary dry eye, trabecular meshwork degeneration, mitochondrial dysfunction and ocular inflammation.

Altogether, the ophthalmic composition of the invention outperforms prior art compositions by adequately balancing moisturizing with remanence, lack of discomfort and lack of undesired side effects. An ophthalmic composition is therefore provided which is effective for the prevention and/or treatment of an ophthalmological pathology, such as dry eye syndrome, Sjogren syndrome, inflammation of the conjunctiva, irritation, itchiness, burning and allergic reactions of the eye, and related conditions, while being highly convenient for the user. The expression "related conditions" in the present application is understood as conditions which are related to a deficient lubrication of the surface of the eye.

A second aspect of the invention thus refers to an ophthalmic composition as defined in the first aspect of the invention for use in prevention and/or treatment of an ophthalmological pathology selected from the group consisting of dry eye syndrome, Sjögren syndrome, inflammation of the conjunctiva, irritation, itchiness, burning and allergic reactions of the eye, and related conditions. This may also be formulated as an ophthalmic composition as defined in the first aspect of the invention for the preparation of a medicament for prevention and/or treatment of an ophthalmological pathology selected from the group consisting of dry eye syndrome, Sjögren syndrome, inflammation of the conjunctiva, irritation, itchiness, burning and allergic reactions of the eye, and related conditions. The invention also refers to a method for prevention and/or treatment of dry eye syndrome, Sjogren syndrome, inflammation of the conjunctiva, irritation, itchiness, burning and allergic reactions of the eye, and related conditions comprising administering a therapeutically effective amount of an ophthalmic composition as defined in the first aspect of the invention, in a subject in need thereof, including a human.

**Brief Description of Drawings**

Figure 1. Synergic effect on water binding capacity for combinations of sodium hyaluronate (HA) and ectoine with or without dexpanthenol. The ectoine content can be calculated as 100% - sodium hyaluronate % - dexpanthenol %.

Figure 2. Dynamic viscosity change as a function of shear ratio for compositions containing sodium hyaluronate with intrinsic viscosity 0.51 m3/kg which relates to the weight average molecular weight (Mw) of about 196 kDa.

Figure 3. Dynamic viscosity change as a function of shear ratio for compositions containing sodium hyaluronate with intrinsic viscosity 0.95 $m^3$/kg which relates to the weight average molecular weight (Mw) of about 410 kDa.

Figure 4. Dynamic viscosity change as a function of shear ratio for compositions containing sodium hyaluronate with intrinsic viscosity 1.00 $m^3$/kg which relates to the weight average molecular weight (Mw) of about 410 - 450 kDa.

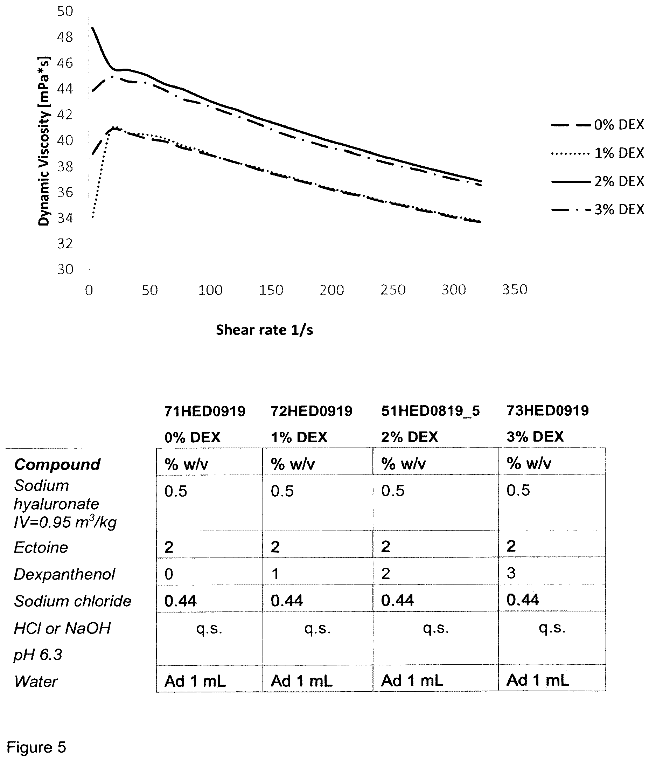

Figure 5. Dynamic viscosity change as a function of shear ratio for compositions containing different concentration of D-panthenol.

Figure 6. Dynamic viscosity drop as measured between maximal and minimal value of the viscosity.

Figure 7. Influence of D-panthenol on surface tension.

Figure 8. Dynamic viscosity for compositions comprising dexpanthenol, sodium hyaluronate and ectoine (Series 67-71HED0919 do not contain dexpanthenol).

Figure 9. Synergic effect on viscosity after dexpanthenol addition.

## Detailed description of the invention

The present application provides an aqueous ophthalmic composition comprising a synergic combination of hyaluronic acid or an ophthalmologically acceptable salt thereof, ectoine or an ophthalmologically acceptable salt or derivative thereof, and a compound selected from the group consisting of panthenol, pantothenic acid, an ophthalmologically acceptable salt of pantothenic acid, and combinations thereof.

Hyaluronic acid (CAS Registry Number: 9004-61-9) is a natural substance which can be found in the eye but also in other parts of the body. It is a long-chain polymer of disaccharide units of glucuronic acid and N-acetylglucosamine linked via alternating $\beta$-(1 →4) and $\beta$-(1→3) glycosidic bonds. Hyaluronic acid can be 25,000 disaccharide repeats in length. Polymers of hyaluronic acid can range in size from 5,000 to 20,000,000 Da in vivo. In the eye it ensures that a uniform, stable and particularly long-term-adhering moisture film is formed on the surface which cannot be rinsed off rapidly.

Several ophthalmologically acceptable salts of hyaluronic acid are known which can be used in the composition of the invention, the most common of which is sodium hyaluronate (herein also termed as HA, PubChem CID: 23663392). The term "ophthalmologically acceptable salts" encompasses any salt formed from ophthalmologically acceptable non-toxic acids including inorganic or organic acids. There is no limitation regarding the salts, except that if used for therapeutic purposes, they must be ophthalmologically acceptable.

The concentration of hyaluronic acid or an ophthalmologically acceptable salt thereof in the composition of the invention is from 0.001 to 2 % by weight. In the present application, "% by weight" means % w/v, i.e. percentage by weight of the ingredient with respect to the total volume of the composition. In other words, x to y % by weight of ingredient A represents x to y grams of ingredient A in 100 ml of the composition. In some embodiments, the composition comprises 0.1 to 0.5 % by weight of hyaluronic acid or an ophthalmologically acceptable salt thereof. In other embodiments, the composition comprises 0.15 to 0.4 % by weight of hyaluronic acid or an ophthalmologically acceptable salt thereof. In other embodiments, the composition comprises 0.3 to 0.4 % by weight of hyaluronic acid or an ophthalmologically acceptable salt thereof. In some embodiments the composition comprises 0.3 % by weight of hyaluronic acid or an ophthalmologically acceptable salt thereof.

Hyaluronic acid and the ophthalmologically acceptable salt thereof may be in the form of cross-linked polymer. Preferably, the polymer is not cross-linked. In addition, the weight average molecular weight (Mw) of the hyaluronic acid or of the ophthalmologically acceptable salt thereof is preferably from 100 to 800 kDa. In some embodiments, the molecular weight of the hyaluronic acid or ophthalmologically acceptable salt thereof is from 150 to 500 kDa. In other embodiments, the molecular weight of the hyaluronic acid or ophthalmologically acceptable salt thereof is from 200 to 450 kDa. The weight average molecular weight of the hyaluronic acid or of the ophthalmologically acceptable salt thereof may be determined by Size extrusion chromatography - multiangle laser scattering (SEC MALS).

In a particular embodiment, the ophthalmologically acceptable salt of hyaluronic acid is selected from the group consisting of sodium hyaluronate, potassium hyaluronate and combinations thereof. In a particular embodiment the ophthalmologically acceptable salt of hyaluronic acid is sodium hyaluronate.

In some embodiments, the composition comprises 1 to 3 % by weight of ectoine or an ophthalmologically acceptable salt or derivative thereof. In some embodiments, the composition comprises 1.5 to 2.5 % by weight of ectoine or an ophthalmologically acceptable salt or derivative thereof. In some embodiments, the composition comprises 2 % by weight of ectoine or an ophthalmologically acceptable salt or derivative thereof.

Ectoines are low molecular cyclic amino acids that function as cell protective agents and are naturally contained in extremophilic microorganisms. According to the prior art ectoine increases water binding to the cells, protect the cell against dehydration and stabilize the proteins in a tear film by exclusion mechanism. Moreover, ectoine by forming clusters with water has profound effect on cell membrane stability. In one embodiment the ectoine present in the composition of the invention is L-ectoine ((S)-2-methyl-1,4,5,6-tetrahydropyrimidine-4-carboxylic acid, CAS number: 96702-03-3; PubChem CID: 126041). In particular embodiment the ophthalmologically acceptable derivative of ectoine is selected from the group consisting of an ophthalmologically acceptable ester of ectoine, hydroxyectoine (CAS number: 165542-15-4; PubChem CID: 12011795) or an ophthalmologically acceptable salt thereof, an ophthalmologically acceptable ester of hydroxyectoine and a combination thereof. Appropriate ophthalmologically acceptable salts are e.g. sodium salts, potassium salts and salts with organic amines, e.g. trometamol or triethylamine. Appropriate esters are those which can be obtained by conversion of the 4-carboxy group with alcohols, in particular straight-chain or branched mono- or bivalent alcohols with 1 to 18 carbon atoms, and/or of the 5-hydroxy group with carboxylic acids, in particular straight-chain or branched-chain mono- or bivalent alkyl carboxylic acids with 2 to 20 carbon atoms, e.g. alkylmonocarboxylic acids, amides as product of reaction with alkylamine where alkyl chain possess up to 18 carbon atoms in linear or branched form, and acid addition salts with inorganic or organic acids. In particular embodiments the composition of the invention comprises an ophthalmologically acceptable salt of L-ectoine, for example sodium salt, potassium salt or a salt with organic amines, e.g. trometamol or triethylamine. In another embodiment the composition of the invention comprises an ophthalmologically acceptable salt of hydroxyectoine, for example sodium salt, potassium salt or a salt with organic amines, e.g. tromethamol or triethylamine.

In some embodiments, the composition comprises 1 to 3 % by weight, or 1.5 to 2.5 % by weight, or 1.5 to 2.5 % by weight, for example 2% by weight, of a compound selected from the group consisting of panthenol, pantothenic acid, an ophthalmologically acceptable salt of pantothenic acid, and combinations thereof.

Panthenol (also called pantothenol) (CAS number: 16485-10-2, PubChem CID: 4678) is the alcohol analog of pantothenic acid (vitamin B5, CAS number: 599-54-2), and is thus a provitamin of B5. In organisms it is quickly oxidized to pantothenic acid. In particular embodiments, the compound is D-panthenol (herein also termed as dexpanthenol or DEX, CAS number: 81-13-0; PubChem CID: 131204). In other particular embodiments the compound is an ophthalmologically acceptable salt of pantothenic acid which can be selected from sodium pantothenate and calcium pantothenate.

The ophthalmic composition of the invention may have a weight ratio of hyaluronic acid or an ophthalmologically acceptable salt thereof, to ectoine or an ophthalmologically acceptable salt or derivative thereof, to the compound selected from panthenol, pantothenic acid, an ophthalmologically acceptable salt of pantothenic acid, and combinations thereof that is from 1:1.5:1.5 to 1:100:100. In particular embodiments the weight ratio is 1:1.5:1.5 to 1:30:30. In other embodiments the weight ratio is 1:2:2 to 1:10:10. In other embodiments the weight ratio is 1:4:4 to 1:8:8, for example 1:6.7:6.7.

The composition according to the invention may further contain an additive selected from the group of nonionic type isotonizing agents, antioxidants and / or buffer systems. According to another particular embodiment, the composition of the invention is an isotonic or hypotonic formulation with an osmolality advantageously between 170 and 350 mOsmol / kg. This characteristic can be obtained, for example, by addition of osmolarity regulating agent such as sodium chloride (NaCl). Thus, in particular embodiments the composition of the invention further comprises sodium chloride. The sodium chloride may be present in the composition from 0.01 to 0.1 % by weight. In some embodiments the composition comprises from 0.03 to 0.06 %, or from 0.04 to 0.05 % by weight of sodium chloride.

Furthermore, the ophthalmological composition may further benefit from comprising at least one buffer system, for example a buffer selected from the group consisting of borate buffer, citrate buffer, phosphate buffer, tris buffer, trometamol/maleic acid. In particular embodiments the composition comprises citrate buffer. However, in some embodiments the composition is devoid of a buffer system.

The pH of the composition of the invention may be from 5 to 9. In particular embodiments, the pH is from 5.5 to 7.5, or from 6 to 7. For example, the pH of the composition of the invention may be 6.2 or 6.3. The pH of the composition may be determined by a pH-meter or a pH strip. In particular the pH ranges given above may be determined by a pH-meter WTW pH 1970i. The preferred pH value may be obtained by addition of pH regulating agent such as hydrochloric acid (HCl) and/or sodium hydroxide (NaOH).

According to a particularly preferred embodiment, the composition according to the invention is devoid of preservatives. By preservative it is understood any substance which can be used as ophthalmological preservative, such as e.g. the preservatives listed further on. Preservatives can damage the precorneal tear film and lead to a reduction in the number of microvilli and microplicae of the surface corneal epithelium cells, which results in irritation and/or inflammation of the eye. By dispensing with preservatives in the solution according to the invention, such irritation and/or inflammation can hence be avoided. In particular the composition of the invention is devoid of benzalkonium chloride, which is the most frequently used preservative in eye drops. The composition of the invention is also devoid of phosphates. According to the literature phosphates increase the risk of corneal tissue calcification leading to burning eye and cornea opacity.

It is also preferred that the ophthalmic composition according to the invention contains no further pharmaceutically active ingredients. In particular, the composition of the invention is devoid of anti-inflammatory agents and/or immune-suppressants. In particular embodiments the composition is devoid of further excipients and carriers. Particular embodiments thus refer to a composition comprising hyaluronic acid or an ophthalmologically acceptable salt thereof, ectoine or an ophthalmologically acceptable derivative thereof, a compound selected from the group consisting of panthenol, pantothenic acid, an ophthalmologically acceptable salt of pantothenic acid, and combinations thereof as the only active ingredients and further may comprise osmolarity regulating agent such as NaCl, pH regulating agent such as HCl and/or NaOH and water. These compositions could also contain a buffer, for example, citric buffer. So in preferred embodiments the composition of current invention is devoid of preservatives, further active agents and further excipients and carriers.

In one of the embodiments the ophthalmic composition preferably comprises sodium hyaluronate, L-ectoine and D-panthenol as the only active ingredients, water and, optionally, an osmolarity regulating agent such as NaCl and/or pH regulating agent such as HCl and/or NaOH. Said composition is preferably devoid of preservatives, further active agents and further excipients and carriers. In one embodiment the composition of the invention consists essentially of sodium hyaluronate, L-ectoine and D-panthenol as the only active ingredients, water and, optionally, an osmolarity regulating agent such as NaCl and/or pH regulating agent such as HCl and/or NaOH. The weight average molecular weight (Mw) of sodium hyaluronate is from 100 to 800 kDa, more preferably from 150 to 500 kDa, most preferably from 200 to 450 kDa.

Advantageously, the composition according to the invention has a dynamic viscosity suitable for the targeted topical ophthalmic application. It has been determined that easy and non-threatening removal through lacrimal duct is better when dynamic viscosity of the ophthalmic solution / tear mixture is less than 50 mPa.s and this is accomplished by the composition of the current invention. Thus, in some embodiments, the composition according to the invention has dynamic viscosity at 20 ° C from 1 to 50 mPa.s. In particular embodiments the viscosity is from 4 to 40 mPa.s, preferably from 8 to 20 mPa.s. These values correspond to the values measured using a BROOKFIELD DV3T rotating viscometer at 20°C.

Surprisingly, the triple combination of hyaluronic acid or an ophthalmologically acceptable salt thereof, ectoine or an ophthalmologically acceptable salt or derivative thereof, and a compound selected from the group consisting of panthenol, pantothenic acid, an ophthalmologically acceptable salt of pantothenic acid, and combinations thereof according to the invention shows a synergistic effect on the viscosity of the composition (see figure 9).

The synergic effects achieved by the triple combination of the invention also have a positive effect on the production costs of the composition. The referred ingredients are expensive raw materials. The synergistic viscosity- and water-binding capacity increase proves to be very advantageous for saving on raw materials and costs. As a result of the synergistic effect, a selected viscosity and water-binding capacity can be produced with a lower amount of raw material than in the case of the ophthalmological compositions from the state of the art, as a result of which cost savings can be made. Additionally, lower amount of active ingredients has the advantage of producing less undesired side effects.

As mentioned above, it may be convenient that the composition of the invention is devoid of further active ingredients. However, in other embodiments, the composition according to the invention may also contain one or more active agents in therapeutically effective amounts. Said active agents may be for example selected from antibiotics, antibacterials, antifungals, antivirals, immunosuppressants, anti-inflammatories or anti-laucomatous agents such as prostaglandins, beta-blockers, inhibitors carbonic anhydrase or alpha-adrenergic agonists. Such an active agent may especially be chosen from the following group: aceclidine, acetazolamide, aciclovir, anecortave, apraclonidine, atropine, azapentacene, azelastine, bacitracin, befunolol, betamethasone, betaxolol, bimatoprost, brimonidine, brinzolamide, carbachol, cardolol, celecoxib, chloramphenicol, chlortetracycline, ciprofloxacin, cromoglycate, cyclopentolate, cyclosporine, dapiprazole, demecarium, dexamethasone, diclofenac, dichlorphenamide, dipivefrin, dorzolamide, echothiophale, emedastine, epinastine, epinephrine, erythromycin, ethoxzolamide, eucatropine, fludrocortisone, fluorometholone, flurbiprofen, fomivirsen, framycetin, ganciclovir, gatifloxacin, gentamycin, homatropine, hydrocortisone, idoxuridine, indomethacin, isoflurophate, ketorolac, ketotifen, latanoprost, levobetaxolol, levobunolol, levocabastine, levofloxacin, lodoxamide, loteprednol, medrysone, methazolamide, metipranolol, moxifloxacin, naphazoline, natamycin, nedocromil, neomycin, norfloxacin , o floxacin, olopatadine, oxymetazoline, pemirolast, pegaptanib, phenylephrine, physostigmine, pilocarpine, pindolol, pirenoxine, polymyxin B, prednisolone, proparacaine, ranibizumab, rimexolone, scopolamine, sezolamide, squalamine, sulfacetamide, suprofen, tetracaine, tetracyclin, tetrahydrozoline, tetryzoline, timolol , tobramycin, travoprost, triamcinulone, trifluoromethazolarnide, trifluridine, trimethoprim, tropicamide, unoprostone, vidarbine, xylometazoline, pharmaceutically acceptable salts, or combinations thereof. Quaternary ammonium compounds, especially benzalkonium chloride or polyquad, optionally in combination with EDTA, are generally used as preservatives. Other conservatives are for example: preservatives based on guanidine (PHMB, chlorhexidine);benzyl alcohol; parabens (methyl, ethyl, propyl, ...); mercury-based preservatives, such as thimerosal; chlorobutanol; - benzethonium chloride; oxidative preservatives (Purite, ...).

The ophthalmic composition according to the invention may be conveniently configured in the form of eyedrops. The composition may be configured in the form of a single-use (single-dose) or multi-dose vial made of, for example, Low-density polyethylene (LDPE), advantageously of EP quality containing no additives. Non-limiting examples of single-use vials (single dose) or multi-dose vial to deliver eye drops without preservatives for several days are Abak®, Comod®, OSD® or Novelia®. Apart from the eyedrops, other non-limiting, dosage forms for the composition of the invention are a cream, a gel, a hydrogel, a lotion, an ointment and a spray, all of which for application to the eyes. When used, excipients and carriers incorporated into the composition of the invention must be ophthalmologically acceptable. "Ophthalmologically acceptable excipients or carriers" refers to ophthalmologically acceptable materials, compositions or vehicles. Each component must be ophthalmologically acceptable in the sense of being compatible with the other ingredients of the ophthalmic composition. It must also be suitable for use in contact with the eye without excessive toxicity, irritation, allergic response, immunogenicity or other problems or complications commensurate with a reasonable benefit/risk ratio.

A second aspect of the invention refers to an ophthalmic composition as above for use in the prevention and/or treatment of an ophthalmological (or ocular) pathology. In particular embodiments the ophthalmological pathology is selected from the group consisting of dry eye syndrome, Sjogren syndrome, inflammation of the conjunctiva, irritation, itchiness, burning and allergic reactions of the eye, and related conditions. In a particular embodiment the ophthalmic pathology is dry eye syndrome.

The second aspect may also be contemplated as hyaluronic acid or an ophthalmologically acceptable salt thereof for use in the prevention and/or treatment of an ophthalmological (or ocular) pathology when used in combination with ectoine or an ophthalmologically acceptable salt or derivative thereof and a compound selected from the group consisting of panthenol, pantothenic acid, an ophthalmologically acceptable salt of pantothenic acid, and combinations thereof. This may also be worded as hyaluronic acid or an ophthalmologically acceptable salt thereof for the preparation of a medicament for the prevention and/or treatment of an ophthalmological (or ocular) pathology when used in combination with ectoine or an ophthalmologically acceptable salt or derivative thereof and a compound selected from the group consisting of panthenol, pantothenic acid, an ophthalmologically acceptable salt of pantothenic acid, and combinations thereof.

The second aspect may also be contemplated as ectoine or an ophthalmologically acceptable salt or derivative thereof for use in prevention and/or treatment of an ophthalmological (or ocular) pathology when used in combination with hyaluronic acid or an ophthalmologically acceptable salt thereof and a compound selected from the group consisting of panthenol, pantothenic acid, an ophthalmologically acceptable salt of pantothenic acid, and combinations thereof. This may also be worded as ectoine or an ophthalmologically acceptable salt or derivative thereof for the preparation of a medicament for prevention and/or treatment of an ophthalmological (or ocular) pathology when used in combination with hyaluronic acid or an ophthalmologically acceptable salt thereof and a compound selected from the group consisting of panthenol, pantothenic acid, an ophthalmologically acceptable salt of pantothenic acid, and combinations thereof.

The second aspect may also be contemplated as a compound selected from the group consisting of panthenol, pantothenic acid, an ophthalmologically acceptable salt of pantothenic acid, and combinations thereof for use in the prevention and/or treatment of an ophthalmological (or ocular) pathology when used in combination with hyaluronic acid or an ophthalmologically acceptable salt thereof and ectoine or an ophthalmologically acceptable salt or derivative thereof. This may also be worded as a compound selected from the group consisting of panthenol, pantothenic acid, an ophthalmologically acceptable salt of pantothenic acid, and combinations thereof for the preparation of a medicament for the prevention and/or treatment of an ophthalmological (or ocular) pathology when used in combination with hyaluronic acid or an ophthalmologically acceptable salt thereof and ectoine or an ophthalmologically acceptable salt or derivative thereof.

The ingredients in the combination may be administered simultaneously, sequentially or separately. Preferably they are administered simultaneously forming part of the same composition.

The composition according to the invention can be used both in humans and in animals. According to a preferred embodiment, this composition is intended for topical application, preferably by instillation at the eye.

The dose is adapted to the severity of the pathology but usually consists in administering in the affected body one to several drops of the composition of the invention per day. The composition of the invention makes it possible to space the administrations, in comparison in particular with a composition not containing the triple combination of ingredients. Thus, a dose of less than 5 drops per day and per eye, or even less than 4, 3, 2 or even 1 single drop per day and by that it can be considered.

The present application also contemplates that the triple combination of the invention as defined above provides a basis for formulating sustained release preparations ("drug delivery system"), for improving bioavailability, remanence and hence the effectiveness of the composition, particularly as described above. Thus, another embodiment refers to a sustained release ophthalmic formulation comprising the composition of the invention as defined above, as well as its use in the prevention and/or treatment of an ophthalmological pathology, in particular dry eye syndrome.

For reasons of completeness, various aspects of the invention are set out in the following numbered embodiments:

Embodiment 1. An ophthalmic composition comprising:

0.001 to 2 % by weight of hyaluronic acid or an ophthalmologically acceptable salt thereof, 0.5 to 5 % by weight of ectoine or an ophthalmologically acceptable salt or derivative thereof,
0.5 to 5 % by weight of a compound selected from the group consisting of panthenol, pantothenic acid, an ophthalmologically acceptable salt of pantothenic acid, and combinations thereof, and
an amount of water up to 100% by weight of the composition.

Embodiment 2. The ophthalmic composition according to embodiment 1, that comprises 0.1 to 0.5 % by weight of hyaluronic acid or an ophthalmologically acceptable salt thereof.

Embodiment 3. The ophthalmic composition according to embodiment 2, that comprises 0.15 to 0.4 % by weight of hyaluronic acid or an ophthalmologically acceptable salt thereof.

Embodiment 4. The ophthalmic composition according to embodiment 3, that comprises 0.3 to 0.4 % by weight of hyaluronic acid or an ophthalmologically acceptable salt thereof.

Embodiment 5. The ophthalmic composition according to embodiment 4, that comprises 0.3 % by weight of hyaluronic acid or an ophthalmologically acceptable salt thereof.

Embodiment 6. The ophthalmic composition according to any of the embodiments 1-5, that comprises 1 to 3 % by weight of ectoine or an ophthalmologically acceptable salt or derivative thereof.

Embodiment 7. The ophthalmic composition according to embodiment 6, that comprises 1.5 to 2.5 % by weight of ectoine or an ophthalmologically acceptable salt or derivative thereof.

Embodiment 8. The ophthalmic composition according to embodiment 7, that comprises 2 % by weight of ectoine or an ophthalmologically acceptable salt or derivative thereof.

Embodiment 9. The ophthalmic composition according to any of the embodiments 1-8, that comprises 1 to 3 % weight of a compound selected from the group consisting of panthenol, pantothenic acid, an ophthalmologically acceptable salt of pantothenic acid, and combinations thereof.

Embodiment 10. The ophthalmic composition according to embodiment 9, that comprises 1.5 to 2.5 % by weight of a compound selected from the group consisting of panthenol, pantothenic acid, an ophthalmologically acceptable salt of pantothenic acid, and combinations thereof.

Embodiment 11. The ophthalmic composition according to embodiment 9, that comprises 2 % by weight of a compound selected from the group consisting of panthenol, pantothenic acid, an ophthalmologically acceptable salt of pantothenic acid, and combinations thereof.

Embodiment 12. The ophthalmic composition according to any of the embodiments 1-11, wherein the weight average molecular weight (Mw) of the hyaluronic acid or ophthalmologically acceptable salt thereof is from 100 to 800 kDa.

Embodiment 13. The ophthalmic composition according to embodiment 12, wherein the weight average molecular weight (Mw) of the hyaluronic acid or ophthalmologically acceptable salt thereof is from 150 to 500 kDa.

Embodiment 14. The ophthalmic composition according to embodiment 13, wherein the weight average molecular weight (Mw) of the hyaluronic acid or ophthalmologically acceptable salt thereof is from 200 to 450 kDa.

Embodiment 15. The ophthalmic composition according to any of the embodiments 1-14, that comprises sodium hyaluronate.

Embodiment 16. The ophthalmic composition according to any of the embodiments 1-15, that contains ectoine or an ophthalmologically acceptable salt thereof, optionally selected from sodium salt, potassium salt and salts with organic amines, optionally selected from tromethamol and triethylamine.

Embodiment 17. The ophthalmic composition according to any of the embodiments 1-16, that contains L-ectoine.

Embodiment 18. The ophthalmic composition according to any of the embodiments 1-17, that contains an ophthalmologically acceptable derivative of ectoine selected from the group consisting of an ophthalmologically acceptable ester of ectoine, hydroxyectoine or an ophthalmologically acceptable salt of hydroxyectoine, an ophthalmologically acceptable ester of hydroxyectoine and a combination thereof.

Embodiment 19. The ophthalmic composition according to any of the embodiments 1-18, that contains D-panthenol.

Embodiment 20. The ophthalmic composition according to any of the embodiments 1-18, that contains an ophthalmologically acceptable salt of pantothenic acid selected from sodium pantothenate and calcium pantothenate.

Embodiment 21. The ophthalmic composition according to any of the embodiments 1-20, wherein the weight ratio of hyaluronic acid or an ophthalmologically acceptable salt thereof, to ectoine or an ophthalmologically acceptable derivative thereof, to the compound selected from panthenol, pantothenic acid, an ophthalmologically acceptable salt of pantothenic acid and combinations thereof is from 1:1.5:1.5 to 1:100:100.

Embodiment 22. The ophthalmic composition according to the preceding embodiment, wherein the weight ratio is 1:1.5:1.5 to 1:30:30.

Embodiment 23. The ophthalmic composition according to the preceding embodiment, wherein the weight ratio is 1:2:2 to 1:10:10.

Embodiment 24. The ophthalmic composition according to the preceding embodiment, wherein the weight ratio is 1:4:4 to 1:8:8.

Embodiment 25. The ophthalmic composition according to the preceding embodiment, wherein the weight ratio is 1:6.7:6.7.

Embodiment 26. The ophthalmic composition according to any of the preceding embodiments, further comprising an osmolarity regulating agent.

Embodiment 27. The ophthalmic composition according to the preceding embodiment, wherein the osmolarity regulating agent is sodium chloride.

Embodiment 28. The ophthalmic composition according to any of the preceding embodiments, further comprising a buffer.

Embodiment 29. The ophthalmic composition according to the preceding embodiment, wherein the buffer is selected from the group consisting of borate buffer, citrate buffer, phosphate buffer, tris buffer, trometamol/maleic acid

Embodiment 30. The ophthalmic composition according to any of the preceding embodiments, whose pH is from 6 to 7.

Embodiment 31. The ophthalmic composition according to the preceding embodiment, whose pH is 6.3.

Embodiment 32. The ophthalmic composition according to any of the embodiments 27-31, that comprises citrate buffer.

Embodiment 33. The ophthalmic composition according to any of the preceding embodiments, that comprises pH regulating agent which is selected from HCl and/or NaOH.

Embodiment 34. The ophthalmic composition according to any of the preceding embodiments, which is devoid of preservatives and/or of any further pharmaceutically active ingredients.

Embodiment 35. The ophthalmic composition according to any of the preceding embodiments, that is devoid of preservatives.

Embodiment 36. The ophthalmic composition according to any of the preceding embodiments, that is devoid of any further excipients or carriers.

Embodiment 37. The ophthalmic composition according to any of the preceding embodiments, that has dynamic viscosity measured by Brookfield method at 20 ° C from 1 to 50 mPa•s.

Embodiment 38. The ophthalmic composition according to the preceding embodiment, that has dynamic viscosity measured by Brookfield method at 20 ° C from 4 to 40 mPa•s.

Embodiment 39. The ophthalmic composition according to the preceding embodiment, that has dynamic viscosity measured by Brookfield method at 20 ° C from 8 to 20 mPa•s.

Embodiment 40. The ophthalmic composition according to any of the preceding embodiments, that is configured in the form of eyedrops.

Embodiment 41. A composition as defined in any of the preceding embodiments for use in the prevention and/or treatment of an ophthalmological pathology.

Embodiment 42. The composition for use according to the preceding embodiment, wherein the ophthalmological pathology is selected from the group consisting of dry eye syndrome, Sjogren syndrome, inflammation of the conjunctiva, irritation, itchiness, burning and allergic reactions of the eye, and related conditions.

Embodiment 43. The composition for use according to the preceding embodiment, wherein the ophthalmological pathology is dry eye syndrome.

Throughout the description and claims the word "comprise" and variations of the word, are not intended to exclude other technical features, additives, components, or steps. Furthermore, the word "comprise" encompasses the case of "consisting essentially of" and/or "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

**Examples**

Materials

Experiments were run with L-ectoine, dexpanthenol and sodium hyaluronate. Sodium hyaluronate with the following weight average molecular weight (Mw) was used:

*Table 1. Mw for a given range of intrinsic viscosity of HA*

| Intrinsic viscosity [m3/kg] | Mean Molecular Weight [kDa] |
|---|---|
| 0.51 | 196 |

(continued)

| Intrinsic viscosity [m3/kg] | Mean Molecular Weight [kDa] |
|---|---|
| 0.95 | 410 |
| 1.00 | 485 |

Formulations

In total, aqueous compositions with different concentration of sodium hyaluronate and ectoine (comparative compositions) and sodium hyaluronate, ectoine and dexpanthenol were prepared. Some compositions also contained 0.44 NaCl and were adjusted to pH 6.3 with HCl or NaOH as required As non-limiting examples, the preparation of two said compositions are described in detail below.

A composition containing 0.1% or 0.2% or 0.3% or 0.4% or 0.5% sodium hyaluronate, 2% ectoine and 2% dexpanthenol was prepared as follows: firstly, in the beaker 44 mg of sodium chloride is dissolved in approx. 70 mL deionized water. After complete dissolution, 2007.0 mg of ectoine is added and stirred as long as clear solution is obtained. Then 102 mg or 204 mg or 306 mg or 408 mg or 510 mg of sodium hyaluronate is added slowly and agitated at elevated temperature (50 °C) until clear solution is obtained. In the second beaker 2013 mg of dexpanthenol is added to approx. 10 mL of deionized water and stirred until clear solution. In the next step the solution with dexpanthenol is added to the first beaker. Thereafter solution is filled up with deionized water to 300 mL, and the pH is adjusted to 6.3 by using 0.1 M HCl.

A composition containing 0.1% or 0.2% or 0.3% or 0.4% or 0.5% sodium hyaluronate, 2% ectoine and 2% dexpanthenol: firstly, in the beaker 501 mg of sodium citrate and 52 mg of citric acid are dissolved stepwise in approx. 200 mL deionized water. After complete dissolution, 6000.0 mg of ectoine is added and stirred as long as clear solution is obtained. Then 919 mg of sodium hyaluronate is added slowly and agitated at elevated temperature (35 °C) until clear solution is obtained. In the second beaker 6041 mg of dexpanthenol is added to approx. 30 mL of deionized water and stirred until clear solution. In the next step the solution with dexpanthenol is added to the first beaker. The mixture is stirred and thereafter solution is filled up with deionized water to 300 mL.

Water binding capacity

The water binding capacity (WBK) was determined as the maximum amount of water imbibed by the sample and retained under low-speed centrifugation (centrifuge Heraeus Sepatech model Labofuge Ae). This effect was measured by the gravimetric method employing a microbalance with d=0.001 mg (balance Sartorius model MSA66P) as describe below.

Sodium hyaluronate, L-ectoine and dexpanthenol were weighted into pre-weighed Eppendorf vessels at the different proportions as shown for the combinations in figures 1 (total weight of the mixtures was 50 mg in all cases). The initial total weight of the mixture was noted ($w_0$). Subsequently, distilled water in small drops was added, stirred with a needle after each addition until mixture was thoroughly wetted without the effect of flowing. Next, water was added to the sample without mixing until a small water excess was present in the upper part of the mixture. Then the sample was centrifuged at 4000 rpm for 5 minutes at ambient temperature. After centrifugation, the slight amount of a supernatant was discarded. Next, the total weight of the mixture was determined after removing the supernatant (wi).

The measured water binding capacity (WBK) was determined with the following formula:

$$WBK\ (gravimetric)\ in\ \% = \frac{(w_1 - w_0) \cdot 100\ \%}{w_0}$$

where:

"wo" = weight of initially total mass of the mixture;
"$w_1$" = weight of total mass of the mixture after centrifugation and without supernatant.

Also, WBK in % was measured for each pure ingredient as described above (pure sodium hyaluronate, pure ectoine, pure dexpanthenol, each of them weighted in quantity of 50 mg).

Next, the theoretical WBK of the combinations was calculated as the sum of the WBK% of pure ingredients in the proportions the ingredients are present in the mixture or as the sum of the WBK% of pure dexpanthenol and the WBK% of the combinations of sodium hyaluronate and L-ectoine. This theoretical WBK is the water binding capacity which would have been expected in the case of a purely additive effect.

The deviation of the measured WBK from the calculated theoretical WBK reflects the synergistic effect, which can be calculated according to following equation:

$$Synergy = \frac{WBK\ measured * 100\%}{WBK\ theoretical}$$

Otherwise expressed, the synergistic effect indicates how much one can decrease the concentration of sodium hyaluronate in the combination to maintain the same level of WBK. The synergistic effect can be seen on Figure 1.

Figure 1 shows the synergic effect in WBK when increasing the concentration of dexpanthenol for 5, 10 and 20% sodium hyaluronate concentrations and different concentrations of ectoine. The ectoine content can be calculated as 100% minus sodium hyaluronate % minus dexpanthenol% (100 - HA% - DEX%). It was observed that the addition of dexpanthenol increased the amount of bound water compared with the composition which only contained sodium hyaluronate and ectoine (Figure 1). The effect became more significant when the concentration of sodium hyaluronate decreased below 20% w/v.

Viscosity drop vs shear ratio

Dynamic viscosity was determined with a Brookfield LV DV3T viscometer. The samples were taken directly to viscosity measurements without any dissolution. The exact sample volume of 6.7mL was poured into the chamber tube surrounded by water jacket, a SC4-18 spindle was placed in the sample and analysis started when the temperature of the sample reached $20.0 \pm 0.2°C$. The data were gathered within a range of spindle rotation 2 - 250 rpm.

The composition of the invention is characterized by an increased dynamic viscosity. If applied eye drops have a dynamic viscosity within the range of physiological tears, they are eliminated within a few minutes. Rising the viscosity of installed eye drops has the advantage of prolonging the time of removing the drops form ocular surface. Figure 8 shows effect of dexpanthenol addition on increasing the dynamic viscosity of the compositions according to the invention.

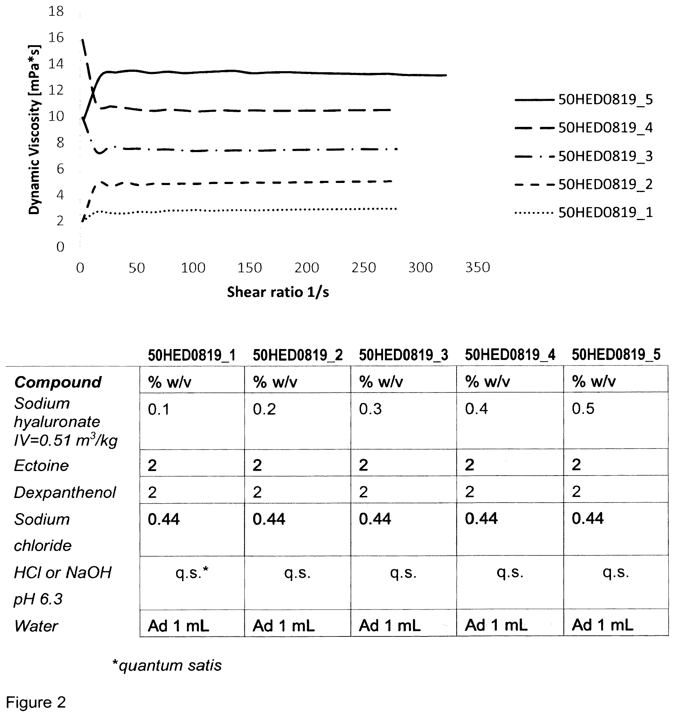

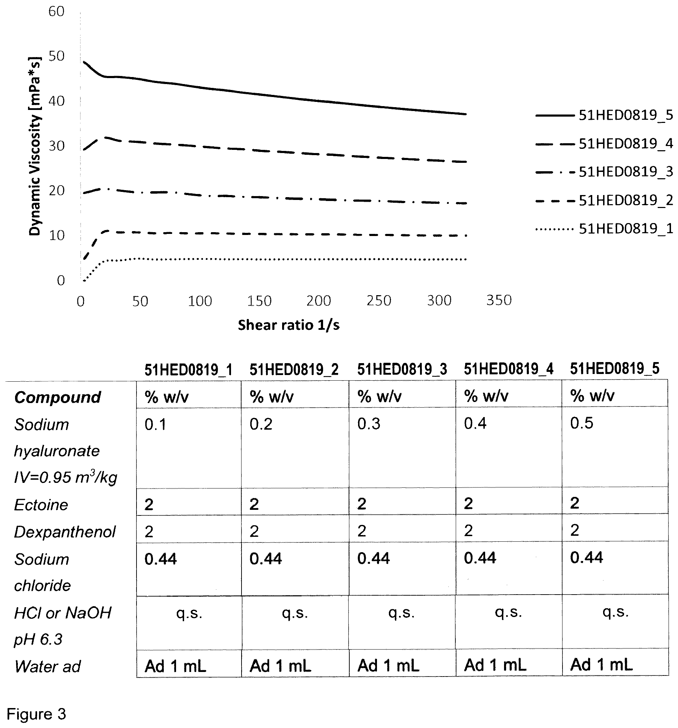

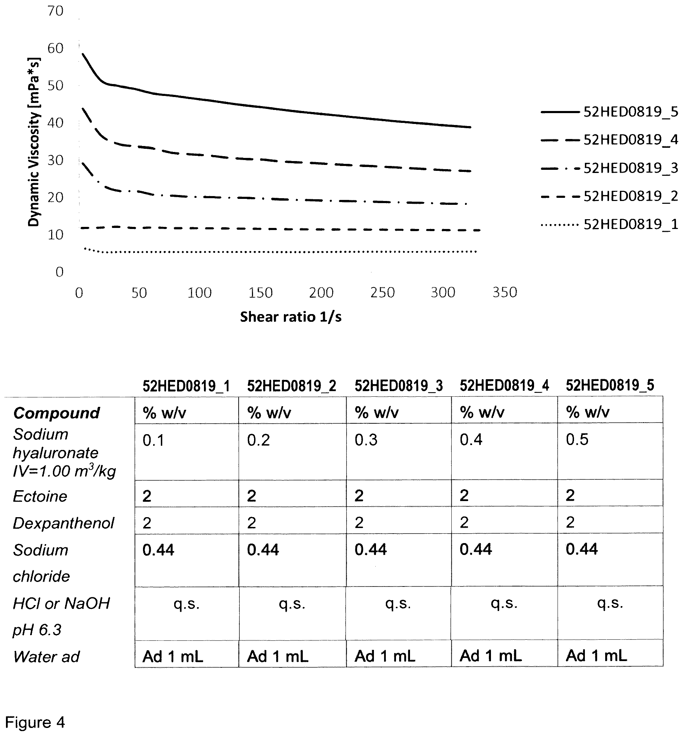

Secondly, the dynamic viscosity of tears decreases while blinking because of very high shear stress generated by eye lids, what prevents the eye from damage. From an eye safety point of view, the velocity of dynamic viscosity drop is an important parameter. The faster the dynamic viscosity drops while blinking, the safer the eye drops are and less potentially damaging to corneal surface. Figure 2, 3 and 4 show how the hyaluronic acid chain length influences the dynamic viscosity as a function of shear ratio. It is noticeable that all formulation with hyaluronic acid concentration equal or below 0.2% regardless of tested molecular weight behave as Newtonian liquids within analyzed shear ratio range (0-250 rpm). It can be observed in Figure 8 that all compositions comprising 2% D-panthenol showed higher viscosities than the same formulations without dexpanthenol. The synergic effect of addition of dexpanthenol to compositions comprising sodium hyaluronate and L-ectoine was also studied (compositions and results are shown in figure 9). It was observed that the composition with dexpanthenol has a higher viscosity than what would have been expected according to the theoretical addition of the viscosities of the compositions containing dexpanthenol (no sodium hyaluronate or ectoine) and compositions containing sodium hyaluronate and ectoine (no dexpanthenol). The highest synergy is observed for 2% dexpanthenol (390.5%, Figure 9).

Moreover the velocity of viscosity drop when the shear ratio increases depends on the concentration of dexpanthenol. It is significant that the highest viscosity drop measured as a difference between maximal and minimal viscosity value (within shear ratio range) can be observed for 2% dexpanthenol ($\Delta\mu$ = 8.66 mPa*s, Figure 6).

Surface tension

Surface tension is a very important parameter in terms of wettability of the cornea surface. The surface tension should be higher than gravitational forces to stick to and spread across corneal surface. Tears of patients suffering from dry eye syndrome have usually higher surface tension than those from healthy individuals.

The aim of these experiments was to determine the influence of the D-panthenol addition on changes in the surface tension. The surface tension was measured in the sitting drop using Wilhelmy method and the solutions were taken directly to analysis without dissolution. It was observed that addition of dexpanthenol indeed decreased the surface tension of the compositions of the invention (as can be seen in Figure 7). The surface tension was measured for a dexpanthenol concentration range 0 - 3% using tensiometer Krüss K20.

**Documents cited in the application**

Wegener, A. R., Meyer, L. M. & Schönfeld, C. L. Effect of viscous agents on corneal density in dry eye disease. J. Ocul. Pharmacol. Ther. 31, 504-508 (2015).

Creech JL, Do LT, Fatt I, Radke CJ. In vivo tear-film thickness determination and implications for tear-film stability. Curr Eye Res 1998; 17: 1058-1066.

J. M. Tiffany, N. Winter & G Bliss (1989) Tear film stability and tear surface tension, Current Eye Research, 8:5, 507-515.

**Claims**

1. An ophthalmic composition comprising:

   0.001 to 2 % by weight of hyaluronic acid or an ophthalmologically acceptable salt thereof,
   0.5 to 5 % by weight of ectoine or an ophthalmologically acceptable salt or derivative thereof,
   0.5 to 5 % by weight of a compound selected from the group consisting of panthenol, pantothenic acid, an ophthalmologically acceptable salt of pantothenic acid, and combinations thereof, and
   an amount of water up to 100% by weight of the composition.

**2.** The ophthalmic composition according to claim 1, wherein the composition comprises:

0.15 to 0.4 % by weight of hyaluronic acid or an ophthalmologically acceptable salt thereof,

1 to 3 % by weight of ectoine or an ophthalmologically acceptable salt or derivative thereof,

1 to 3 % by weight of a compound selected from the group consisting of panthenol, pantothenic acid, an ophthalmologically acceptable salt of pantothenic acid, and combinations thereof, and

an amount of water up to 100% by weight of the composition.

**3.** The ophthalmic composition according to any of the claims 1-2, wherein the weight average molecular weight (Mw) of the hyaluronic acid or of the ophthalmologically acceptable salt thereof is from 100 to 800 kDa.

**4.** The ophthalmic composition according to claim 3, wherein the weight average molecular weight (Mw) of the hyaluronic acid or ophthalmologically acceptable salt thereof is from 150 to 500 kDa.

**5.** The ophthalmic composition according to any of the claims 1-4, wherein the ophthalmologically acceptable derivative of ectoine is selected from the group consisting of an ophthalmologically acceptable ester of ectoine, hydroxyectoine or an ophthalmologically acceptable salt of hydroxyectoine, an ophthalmologically acceptable ester of hydroxyectoine, and a combination thereof.

**6.** The ophthalmic composition according to any of the claims 1-5, wherein the composition comprises sodium hyaluronate, L-ectoine, and D-panthenol.

**7.** The ophthalmic composition according to any of the claims 1-6, wherein the weight ratio of hyaluronic acid or an ophthalmologically acceptable salt thereof, to ectoine or an ophthalmologically acceptable derivative thereof, to the compound selected from panthenol, pantothenic acid, an ophthalmologically acceptable salt of pantothenic acid, and combinations thereof is from 1:1.5:1.5 to 1:30:30.

**8.** The ophthalmic composition according to claim 7, wherein the weight ratio is from 1:2:2 to 1:10:10.

**9.** The ophthalmic composition according to any of the claims 1-8, wherein the composition further comprises sodium chloride.

**10.** The ophthalmic composition according to any of the claims 1-9, wherein the composition is devoid of preservatives and/or of any further pharmaceutically active ingredient.

**11.** The ophthalmic composition according to any of the claims 1-10, wherein the composition has pH from 5 to 7.5.

**12.** The ophthalmic composition according to any of the claims 1-11, wherein the composition has dynamic viscosity as measured by the Brookfield method at 20°C from 1 to 50 mPa.s.

**13.** The ophthalmic composition according to any of the claims 1-12, wherein the composition is in the form of eyedrops.

**14.** A composition as defined in any of the preceding claims for use in the prevention and/or treatment of an ophthalmological pathology selected from the group consisting of dry eye syndrome, Sjogren syndrome, inflammation of the conjunctiva, irritation, itchiness, burning and allergic reactions of the eye, and related conditions.

**15.** The composition for use according to claim 14, wherein the ophthalmological pathology is dry eye syndrome.

Figure 1

| | 50HED0819_1 | 50HED0819_2 | 50HED0819_3 | 50HED0819_4 | 50HED0819_5 |
|---|---|---|---|---|---|
| **Compound** | % w/v | % w/v | % w/v | % w/v | % w/v |
| Sodium hyaluronate IV=0.51 m³/kg | 0.1 | 0.2 | 0.3 | 0.4 | 0.5 |
| Ectoine | 2 | 2 | 2 | 2 | 2 |
| Dexpanthenol | 2 | 2 | 2 | 2 | 2 |
| Sodium chloride | 0.44 | 0.44 | 0.44 | 0.44 | 0.44 |
| HCl or NaOH pH 6.3 | q.s.* | q.s. | q.s. | q.s. | q.s. |
| Water | Ad 1 mL | Ad 1 mL | Ad 1 mL | Ad 1 mL | Ad 1 mL |

*quantum satis

Figure 2

| Compound | 51HED0819_1 | 51HED0819_2 | 51HED0819_3 | 51HED0819_4 | 51HED0819_5 |
|---|---|---|---|---|---|
| | % w/v | % w/v | % w/v | % w/v | % w/v |
| Sodium hyaluronate IV=0.95 $m^3$/kg | 0.1 | 0.2 | 0.3 | 0.4 | 0.5 |
| Ectoine | 2 | 2 | 2 | 2 | 2 |
| Dexpanthenol | 2 | 2 | 2 | 2 | 2 |
| Sodium chloride | 0.44 | 0.44 | 0.44 | 0.44 | 0.44 |
| HCl or NaOH pH 6.3 | q.s. | q.s. | q.s. | q.s. | q.s. |
| Water ad | Ad 1 mL | Ad 1 mL | Ad 1 mL | Ad 1 mL | Ad 1 mL |

Figure 3

| Compound | 52HED0819_1 | 52HED0819_2 | 52HED0819_3 | 52HED0819_4 | 52HED0819_5 |
|---|---|---|---|---|---|
| | % w/v | % w/v | % w/v | % w/v | % w/v |
| Sodium hyaluronate IV=1.00 m³/kg | 0.1 | 0.2 | 0.3 | 0.4 | 0.5 |
| Ectoine | 2 | 2 | 2 | 2 | 2 |
| Dexpanthenol | 2 | 2 | 2 | 2 | 2 |
| Sodium chloride | 0.44 | 0.44 | 0.44 | 0.44 | 0.44 |
| HCl or NaOH pH 6.3 | q.s. | q.s. | q.s. | q.s. | q.s. |
| Water ad | Ad 1 mL | Ad 1 mL | Ad 1 mL | Ad 1 mL | Ad 1 mL |

Figure 4

| Compound | 71HED0919 0% DEX % w/v | 72HED0919 1% DEX % w/v | 51HED0819_5 2% DEX % w/v | 73HED0919 3% DEX % w/v |
|---|---|---|---|---|
| Sodium hyaluronate IV=0.95 m³/kg | 0.5 | 0.5 | 0.5 | 0.5 |
| Ectoine | 2 | 2 | 2 | 2 |
| Dexpanthenol | 0 | 1 | 2 | 3 |
| Sodium chloride | 0.44 | 0.44 | 0.44 | 0.44 |
| HCl or NaOH pH 6.3 | q.s. | q.s. | q.s. | q.s. |
| Water | Ad 1 mL | Ad 1 mL | Ad 1 mL | Ad 1 mL |

Figure 5

| | 71HED0919 | 72HED0919 | 51HED0819_5 | 73HED0919 |
| | 0% DEX | 1% DEX | 2% DEX | 3% DEX |
|---|---|---|---|---|
| **Compound** | **% w/v** | **% w/v** | **% w/v** | **% w/v** |
| Sodium hyaluronate IV=0.95 m³/kg | 0.5 | 0.5 | 0.5 | 0.5 |
| Ectoine | 2 | 2 | 2 | 2 |
| Dexpanthenol | 0 | 1 | 2 | 3 |
| Sodium chloride | 0.44 | 0.44 | 0.44 | 0.44 |
| HCl or NaOH pH 6.3 | q.s. | q.s. | q.s. | q.s. |
| Water | Ad 1 mL | Ad 1 mL | Ad 1 mL | Ad 1 mL |
| Viscosity max mPas*s | 40.84 | 40.84 | 48.74 | 44.93 |
| Viscosity min mPa*s | 33.64 | 33.69 | 36.81 | 36.5 |

Figure 6

Influnce of dexpanthenol on surface tension in
compositions 71HED0919, 72HED0919,
51HED0819_5 and 73HED0919

| Compound | 71HED0919 | 72HED0919 | 51HED0819_5 | 73HED0919 |
|---|---|---|---|---|
| | % w/v | % w/v | % w/v | % w/v |
| Sodium hyaluronate IV=0.95 m³/kg | 0.5 | 0.5 | 0.5 | 0.5 |
| Ectoine | 2 | 2 | 2 | 2 |
| Dexpanthenol | 0 | 1 | 2 | 3 |
| Sodium chloride | 0.44 | 0.44 | 0.44 | 0.44 |
| HCl or NaOH pH 6.3 | q.s. | q.s. | q.s. | q.s. |
| Water | Ad 1 mL | Ad 1 mL | Ad 1 mL | Ad 1 mL |

Figure 7

Figure 8

Synergic effect on viscosity for addition of dexpanthenol
in compositions 72HED091, 51HED0819_5 and 73HED0919

| Compound | 87HED1119 | 86HED1119 | 87HED1119 | 71HED0919 | 72HED0919 | 51HED0819_5 | 73HED0919 |
|---|---|---|---|---|---|---|---|
| | % w/v | % w/v | % w/v | % w/v | % w/v | % w/v | % w/v |
| Sodium hyaluronate IV=0.95 m³/kg | 0 | 0 | 0 | 0.5 | 0.5 | 0.5 | 0.5 |
| Ectoine | 0 | 0 | 0 | 2 | 2 | 2 | 2 |
| Dexpanthenol | 1 | 2 | 3 | 0 | 1 | 2 | 3 |
| Sodium chloride | 0.44 | 0.44 | 0.44 | 0.44 | 0.44 | 0.44 | 0.44 |
| HCl or NaOH pH 6.3 | q.s. | q.s. | q.s. | q.s. | q.s. | q.s. | q.s. |
| Water | Ad 1 mL | Ad 1 mL | Ad 1 mL | Ad 1 mL | Ad 1 mL | Ad 1 mL | Ad 1 mL |

Figure 9

Europäisches
Patentamt

European
Patent Office

Office européen
des brevets

**EUROPEAN SEARCH REPORT**

Application Number

EP 19 46 0062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 108 686 097 A (ZHEJIANG ZHICUITANG BIOTECHNOLOGY CO LTD) 23 October 2018 (2018-10-23) * Formulations 3, 5-7 in Tables 1-2; claims 1-9; example 3 * * paragraph [0002] * | 1,8,10, 14,15 | INV. A61K31/164 A61K31/197 A61K31/505 A61K31/728 A61K9/00 A61K9/08 A61K47/02 A61P27/04 |
| X | DE 10 2011 122236 A1 (KLOSTERFRAU MCM VETRIEB GMBH [DE]) 13 June 2013 (2013-06-13) * paragraphs [0084] - [0085]; claims 1-20; example C' * * paragraphs [0094] - [0100] * | 1,6 | |
| Y | US 2019/099441 A1 (MAHLER MARKUS [DE] ET AL) 4 April 2019 (2019-04-04) * abstract; claims 1-20 * * paragraph [0051] - paragraph [0090] * | 1-15 | |
| Y | US 2017/173012 A1 (BILSTEIN ANDREAS [DE] ET AL) 22 June 2017 (2017-06-22) * abstract; claims 1-13; examples 1-3 * * exeplary embodiments 1-2; paragraph [0025] * | 1-15 | TECHNICAL FIELDS SEARCHED (IPC) A61K A61P |
| Y | EP 0 698 388 A1 (MEDIDOM LAB [CH]) 28 February 1996 (1996-02-28) * abstract; claims 1-16; examples 1-3 * | 1-15 | |
| Y | EP 3 412 276 A2 (OMNIVISION GMBH [DE]) 12 December 2018 (2018-12-12) * abstract; claims 1-15; examples 1-2, 8-9 * * paragraph [0035] * | 1-15 | |

-/--

The present search report has been drawn up for all claims

| Place of search | Date of completion of the search | Examiner |
|---|---|---|
| Munich | 16 June 2020 | Madalinska, K |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 2

Europäisches
Patentamt

European
Patent Office

Office européen
des brevets

**EUROPEAN SEARCH REPORT**

Application Number

EP 19 46 0062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2018 115119 A (ROHTO PHARMA) 26 July 2018 (2018-07-26) * Formulations 1-24; claims 1-7; tables 2-3 * ----- | 1-15 | |
| Y | US 2005/164979 A1 (GROSS DOROTHEA [DE] ET AL) 28 July 2005 (2005-07-28) * abstract; claims 1-10; example 1 * ----- | 1-15 | |
| Y | RU 2 679 319 C1 (MARKOV ILYA ALEKSANDROVICH [RU]) 7 February 2019 (2019-02-07) * claims 1-2; examples 1-11 * ----- | 1-15 | |
| Y | WO 2012/000055 A1 (HOLDEN BRIEN VISION INST [AU]; HOLDEN BRIEN ANTHONY [AU]) 5 January 2012 (2012-01-05) * abstract; claims 1-14; examples 1-2 * ----- | 1-15 | |
| A | WO 2013/097911 A1 (KLOSTERFRAU MCM VETRIEB GMBH [DE]; GREVE HARALD [DE]) 4 July 2013 (2013-07-04) * examples 9-10 * * page 20, lines 1-3 * ----- | 1-15 | TECHNICAL FIELDS SEARCHED (IPC) |

The present search report has been drawn up for all claims

| Place of search | Date of completion of the search | Examiner |
|---|---|---|
| Munich | 16 June 2020 | Madalinska, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 46 0062

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2020

| Patent document cited in search report | | Publication date | Patent family member(s) | | Publication date |
|---|---|---|---|---|---|
| CN 108686097 | A | 23-10-2018 | NONE | | |
| DE 102011122236 | A1 | 13-06-2013 | DE 102011122236 | A1 | 13-06-2013 |
| | | | DE 202011109558 | U1 | 12-12-2012 |
| US 2019099441 | A1 | 04-04-2019 | AU 2017231697 | A1 | 13-09-2018 |
| | | | BR 112018067995 | A2 | 15-01-2019 |
| | | | CA 3015592 | A1 | 14-09-2017 |
| | | | CN 108778250 | A | 09-11-2018 |
| | | | DE 102016203696 | A1 | 07-09-2017 |
| | | | EP 3426226 | A1 | 16-01-2019 |
| | | | JP 2019511494 | A | 25-04-2019 |
| | | | KR 20180117635 | A | 29-10-2018 |
| | | | RU 2018130713 | A | 08-04-2020 |
| | | | US 2019099441 | A1 | 04-04-2019 |
| | | | WO 2017153415 | A1 | 14-09-2017 |
| US 2017173012 | A1 | 22-06-2017 | CA 2949203 | A1 | 26-11-2015 |
| | | | CN 106456537 | A | 22-02-2017 |
| | | | DE 102014007423 | A1 | 26-11-2015 |
| | | | EP 3145511 | A1 | 29-03-2017 |
| | | | JP 2017516770 | A | 22-06-2017 |
| | | | US 2017173012 | A1 | 22-06-2017 |
| | | | WO 2015177353 | A1 | 26-11-2015 |
| EP 0698388 | A1 | 28-02-1996 | AT 187637 | T | 15-01-2000 |
| | | | CA 2154533 | A1 | 26-01-1996 |
| | | | CZ 287223 | B6 | 11-10-2000 |
| | | | DE 69513902 | T2 | 18-05-2000 |
| | | | DK 0698388 | T3 | 08-05-2000 |
| | | | EP 0698388 | A1 | 28-02-1996 |
| | | | ES 2142976 | T3 | 01-05-2000 |
| | | | GR 3032958 | T3 | 31-07-2000 |
| | | | IT RM940485 | A1 | 25-01-1996 |
| | | | JP 3795105 | B2 | 12-07-2006 |
| | | | JP H08169835 | A | 02-07-1996 |
| | | | KR 960003731 | A | 23-02-1996 |
| | | | PL 309753 | A1 | 05-02-1996 |
| | | | PT 698388 | E | 31-05-2000 |
| | | | US 5770628 | A | 23-06-1998 |
| | | | ZA 9506165 | B | 07-03-1996 |
| EP 3412276 | A2 | 12-12-2018 | NONE | | |
| JP 2018115119 | A | 26-07-2018 | NONE | | |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                    EP 19 46 0062

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2020

| Patent document cited in search report | | | Publication date | Patent family member(s) | | Publication date |
|---|---|---|---|---|---|---|
| US 2005164979 | A1 | | 28-07-2005 | AT | 300306 T | 15-08-2005 |
| | | | | AU | 2002360895 A1 | 23-06-2003 |
| | | | | CA | 2468771 A1 | 19-06-2003 |
| | | | | CN | 1602198 A | 30-03-2005 |
| | | | | DE | 10161110 A1 | 26-06-2003 |
| | | | | DE | 50203788 D1 | 01-09-2005 |
| | | | | EP | 1455803 A1 | 15-09-2004 |
| | | | | ES | 2244831 T3 | 16-12-2005 |
| | | | | JP | 3994089 B2 | 17-10-2007 |
| | | | | JP | 2006501133 A | 12-01-2006 |
| | | | | MX | PA04005387 A | 23-03-2005 |
| | | | | US | 2005164979 A1 | 28-07-2005 |
| | | | | WO | 03049747 A1 | 19-06-2003 |
| RU 2679319 | C1 | | 07-02-2019 | | ----------- | |
| WO 2012000055 | A1 | | 05-01-2012 | AU | 2011274245 A1 | 10-01-2013 |
| | | | | CA | 2803935 A1 | 05-01-2012 |
| | | | | CN | 103037858 A | 10-04-2013 |
| | | | | EP | 2588096 A1 | 08-05-2013 |
| | | | | JP | 2013534527 A | 05-09-2013 |
| | | | | SG | 186782 A1 | 28-02-2013 |
| | | | | TW | 201206418 A | 16-02-2012 |
| | | | | US | 2013102679 A1 | 25-04-2013 |
| | | | | US | 2016074345 A1 | 17-03-2016 |
| | | | | WO | 2012000055 A1 | 05-01-2012 |
| WO 2013097911 | A1 | | 04-07-2013 | DE | 102012005452 A1 | 04-07-2013 |
| | | | | DE | 202012002792 U1 | 03-01-2013 |
| | | | | DK | 2766008 T3 | 13-02-2017 |
| | | | | EA | 201491300 A1 | 30-10-2014 |
| | | | | EP | 2766008 A1 | 20-08-2014 |
| | | | | EP | 2818163 A1 | 31-12-2014 |
| | | | | ES | 2612652 T3 | 18-05-2017 |
| | | | | GE | P201706609 B | 25-01-2017 |
| | | | | PL | 2766008 T3 | 31-05-2017 |
| | | | | UA | 113749 C2 | 10-03-2017 |
| | | | | WO | 2013097911 A1 | 04-07-2013 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WEGENER, A. R. ; MEYER, L. M. ; SCHONFELD, C. L.** Effect of viscous agents on corneal density in dry eye disease. *J. Ocul. Pharmacol. Ther.,* 2015, vol. 31, 504-508
- **CREECH JL ; DO LT ; FATT I ; RADKE CJ.** In vivo tear-film thickness determination and implications for tear-film stability. *Curr Eye Res,* 1998, vol. 17, 1058-1066
- **J. M. TIFFANY ; N. WINTER ; G BLISS.** Tear film stability and tear surface tension. *Current Eye Research,* 1989, vol. 8 (5), 507-515
- *CHEMICAL ABSTRACTS,* 9004-61-9
- *CHEMICAL ABSTRACTS,* 96702-03-3
- **WEGENER, A. R. ; MEYER, L. M. ; SCHÖNFELD, C. L.** Effect of viscous agents on corneal density in dry eye disease. *J. Ocul. Pharmacol. Ther.,* 2015, vol. 31, 504-508